# EUROPEAN PATENT SPECIFICATION

(11) **EP 2 531 448 B1**
(45) Date of publication and mention of the grant of the patent: **04.07.2018**
(21) Application number: 11703649.1
(22) Date of filing: 04.02.2011
(51) Int. Cl.: C01G 9/02, A61K 8/58, A61Q 17/04, A61K 8/02, A61K 8/04, B82Y 30/00, C09C 1/04

(54) **ZnO NANOPARTICLES WITH PRISMATIC SHAPE AND THE EFFECT ON OPTICAL PROPERTIES**
ZNO-NANOPARTIKEL MIT PRISMATISCHER FORM UND DER EFFEKT AUF OPTISCHE EIGENSCHAFTEN
NANOPARTICULES DE ZNO DE FORME PRISMATIQUE ET LEUR EFFET SUR DES PROPRIÉTÉS OPTIQUES

(30) Priority: 18.02.2010 US 282480 P; 05.02.2010 EP 10152770
(43) Date of publication of application: 12.12.2012
(73) Proprietor: Grillo Zinkoxid GmbH, 38664 Goslar (DE)
(72) Inventor: POLARZ, Sebastian, 78462 Konstanz (DE); LIZANDARA-PUEYO, Carlos, 78462 Konstanz (DE)
(74) Representative: dompatent von Kreisler Selting Werner - Partnerschaft von Patent- und Rechtsanwälten mbB
(86) International application number: PCT/EP2011/051650
(87) International publication number: WO 2011/095589

(56) References cited:
- MYRTIL L. KAHN, ARNAUD GLARIA, CAROLE PAGES, MIGUEL MONGE, LÉNA SAINT MACARY, ANDRÉ MAISONNAT AND BRUNO CHAUDRET: "Organometallic chemistry: an alternative approach towards metal oxide nanoparticles", J. MATER. CHEM., vol. 19, 5 March 2009 (2009-03-05), pages 4044-4060, XP002573558,
- S. POLARZ, R. REGENSPURGER, J. HARTMANN: "Self-Assembly of Methylzinc-Polyethylene Glycol Amphiphiles and Their Application to Materials Synthesis", ANGEW. CHEM. INTERNAT. ED., vol. 46, no. 14, 2007, pages 2426-2430, XP002573561,
- C. LIZANDARA-PUEYO, M. W. E. VAN DEN BERG, A. DE TONI, T. GOES, S. POLARZ: "Nucleation and Growth of ZnO in Organic Solvents - an in Situ Study", J. AM. CHEM. SOC., vol. 130, 2008, pages 16601-16610, XP002573560, cited in the application
- REVATHI BACSA, YOLANDE KIHN, MARC VERELST, JEANNETTE DEXPERT, WOLFGANG BACSA, PHILIPPE SERP: "Large scale synthesis of zinc oxide nanorods by homogeneous chemical vapour deposition and their characterisation", SURFACE AND COATINGS TECHNOLOGY, vol. 201, 2007, pages 9200-9204, XP002573559,

## Description

The present invention pertains to a process for manufacturing ZnO, the ZnO particles with anisotropic shape obtained by the process of the invention, the effect of the particle shape on optical properties and the use of the ZnO particles.

Zinc oxide is a colourless wide-band gap II/VI semiconductor which possesses a band gap of 3.3 eV (λ(gap) ≈ 380 nm) in its normal form. Therefore, ZnO absorbs light in the entire UV-B region (λ ≈ 280-315 nm) and well into but not the entire UV-A region (λ ≈ 315- 400 nm).

ZnO is used in sunscreens, ointments, paints and varnishes to protect against sunburn and other damage to the skin caused by ultraviolet light. It protects paints and varnishes, the painted material or the product itself from damage caused by ultraviolet light (ageing, embrittlement, bleaching).

Myrtil L. Kahn et al. (J. Mater Chem., vol. 19,5 March 2009 (2009-03-05), pages 4044-4060) discloses a method for synthesizing zinc oxide nanoparticles using alkyl zinc compounds, namely Zn (c-C₆H₁₁)₂ complex, as zinc precursor. The organozinc precursor is reacted with water in an organic solvent like toluene in the presence of n-hexadecylamine.

Revathi Bacsa et al. (Surface and Coatings Technology, vol. 201, 2007, pages 9200-9204) discloses zinc oxide nanorods with a hexagonal basis showing ten times higher absorption for UV radiation when compared to commercial nanoscale ZnO powders.

C. Lizandara-Pueyo et al. (J. Am. Chem. Soc., vol. 130, 2008, pages 16601-16610) discloses zinc oxide nanoparticles which can be used as UV-blocker in sun-lotions.

S. Polarz et al. (Angew. Chem. Internat. Ed., vol. 46, no. 14, 2007, pages 2426-2430) discloses mesoporous nanocrystalline zinc oxide materials prepared from dimeric organometallic amphiphiles containing two polymeric (poly-ethylene glycol) groups.

However, the coverage of the entire UV-A region is currently not possible using zinc oxide alone.

One object of the invention is to provide a ZnO which is able to absorb UV also in higher wavelength ranges of the UV-A region.

The process of the invention yields a product which is able to accomplish this object of the invention. The product of the invention is a crystalline ZnO nanoparticle with a prismatic shape having a triangular basis and a special set of surfaces terminating the particles decreasing the band-gap of the materials. The absorption edge of the product of the invention is shifted to higher values, the range of this shift is about 20 nm. The product of the invention yields a material which is suitable for absorption of the entire UV-A region. The ZnO particles of the invention obtainable by the process of the invention can be used in various fields such as cosmetics or other industrial fields.

However, the nanorods described in Myrtil L. Kahn et al. possess a hexagonal basis, in contrast to the particles according to the invention, which have a prismatic shape with a triangular basis. Thus, the special particle shape of the present invention has a major influence on the functional properties of the obtained product resulting in the shift of the absorption edge to lower energies, which has not been described in the prior art. Furthermore, Myrtil L. Kahn et al. use a monomeric zinc compound with two cyclo-hexyl groups attached to zinc which does not represent a single-source precursor that contains all elements necessary for the formation of ZnO, in contrast to the present invention, wherein a tetrameric zinc-oxo compound with alkyl groups R and, at the same time, alkoxide groups R' attached to zinc is used. The latter contains zinc and oxygen in a 1:1 ratio and is a single-source precursor.

The materials described in Revathi Bacsa et al. absorb light below 380 nm unlike the ZnO nanoprisms of the present invention which show a red-shift of the absorption edge up to 400 nm covering the entire UV-A region.

In contrast to the present invention the particles described in C. Lizandara-Pueyo et al. have no special shape and the size of the ZnO crystallites is below 10 nm, which induces the well known quantum size-effect, a blue-shift of the absorption edge (<380 nm; 3.3 eV). This fact makes them unsuitable for absorbing in the entire UV-A region. In addition, it has been described that the particles precipitate, because no colloidal stabilizers have been used. This prohibits to re-disperse the particles in any medium after synthesis. However, for the preparation of a sun-lotion, it is necessary to re-disperse the particles in a complex mixture of compounds (water, oil, etc.).

The present invention uses the tetrameric ('Zn₄O₄') heterocubanes for the preparation of ZnO nanoprisms. Therefore, the precursors used in S. Polarz et al. are significantly different from the precursors used in the present invention. Furthermore, the organometallic amphiphiles described in S. Polarz et al. self-assemble to certain organized structures. The removal of the polymeric chains via calcination leads to the formation of porosity. The ZnO formation occurs via thermal decomposition of the precursor and not by a reaction with water, in contrast to the present invention. An emulsion has not been used at any time in S. Polarz et al. contrary to the present invention. Since the compounds described in S. Polarz et al. are not stable against water, it is impossible to form an emulsion with them.Subject of the present invention is also a process for manufacturing colloidal dispersions of ZnO comprising the steps of:
- providing an emulsion of an aqueous solution, an aprotic non-polar organic solvent which is essentially non-soluble with water, and an emulsifier containing functional groups capable of specific interaction with ZnO surfaces selected from the group consisting of a polyglycerylate;
- adding, by maintaining the emulsion, as organo-metallic precursor, a tetrameric zinc-oxo compound selected from the group consisting of the formula [RZnOR']₄ where R is Me or Et and R' is Me, Et, Pr, Bu, iso-Pr, tert-Bu, or OCH₂CH₂OCH₃, able to form ZnO;
- reacting the mixture for a sufficient time to form ZnO,
- removal of the water and the aprotic non-polar organic solvent upon forming of said ZnO, and
- isolating the reaction product in which nanoparticles of ZnO with prismatic shape having a triangular basis are obtained in a colloidal dispersion.

- Fig. 1:: Schematical representation of the transformation of the 'Zn₄O₄' heterocubane precursors to ZnO.
- Fig. 2:: TGA traces for ZnO samples of the invention.
- Fig. 3:: Particle size distribution function and PXRD data of ZnO of the invention.
- Fig. 4:: STEM image of ZnO of the invention.
- Fig. 5 and Fig. 7:: PXRD pattern of ZnO particles of the invention.
- Fig. 6:: UV/VIS spectra of ZnO particles of the invention.
- Fig. 8:: Warren-Averbach fit for PXRD data
- Fig. 9:: Electron microscopy data.
- Fig. 10:: ¹H-NMR data in the liquid phase.

In one embodiment of the invention the obtained colloidal dispersion is further purified to obtain nanoparticles of ZnO. In another embodiment of the process of the invention the non-polar organic solvent is a saturated, unsaturated or aromatic hydrocarbon, in particular a cyclic hydrocarbon such as cyclohexane.

In a further embodiment of the process the emulsifier contains a head-group in which donor centers (O, N, S, COOH) are present with a distance of about 3.2 Å thus resembling the Zn-Zn distance in the {100} lattice plane of ZnO.
In yet another embodiment of the process of the invention the emulsifier is an emulsifier binding to the {100} lattice plane of Zn, such as a w/o emulsifier having a HLB value of about 3 to 5, in particular a hydrophilic group, like a polyglycerylate on one side and hydrophobic tail on the other side. A suitable emulsifier is, for example, polyglyceryl-3 polyricinoleate. The process of the invention uses a metallo organic ZnO precursor which is soluble in a variety of aprotic, non-polar solvents, and wherein the precursor is able to be converted to ZnO via hydrolysis, and made up according to the fomula RZnOR' where R can be Me or Et and R' can be Me, Et, Pr, Bu, iso-Pr, tert-Bu, or OCH₂CH₂OCH₃. In yet another embodiment of the invention the metallo organic ZnO precursor is methylzinc-isopropoxide heterocubane. In another embodiment of the invention the metallo organic precursor is a single-source precursor.

Furthermore, a subject matter of the invention is a colloidal ZnO with prismatic shape having a triangular basis containing preparation obtainable by the process according to the invention. The ZnO preparation of the invention can be used as UV-A absorbing agent, in particular as UV-protective material for cosmetic and industrial applications.

The method of the invention is suitable for the preparation of anisotropic ZnO nanoparticles. This method combines the advantages of a water in oil emulsion with an organometallic precursor system for the generation of ZnO. The resulting pastes contain colloidal ZnO nanoparticles with prismatic shape having a triangular basis. The maximum ZnO content in these pastes is with 67% remarkable high considering that it is possible to redisperse the particles in a variety of common organic solvents. The prismatic nanoparticles of the invention are terminated with the polar {002} surface perpendicular to the main axis which furnishes them with a certain dipole moment. Furthermore, the material obtainable by the present invention provides a red-shift of the band-gap energy depending on the length of the nanoprisms. A maximum shift of ≈+20nm was realized for the sample containing the maximum ZnO. The red-shift is sufficient to cover the entire UV-A range of the electromagnetic spectrum. Both properties, good ability for redispersion and the described special optical properties render these materials promising candidates for the all- mineral UV-protection in sun-lotions.

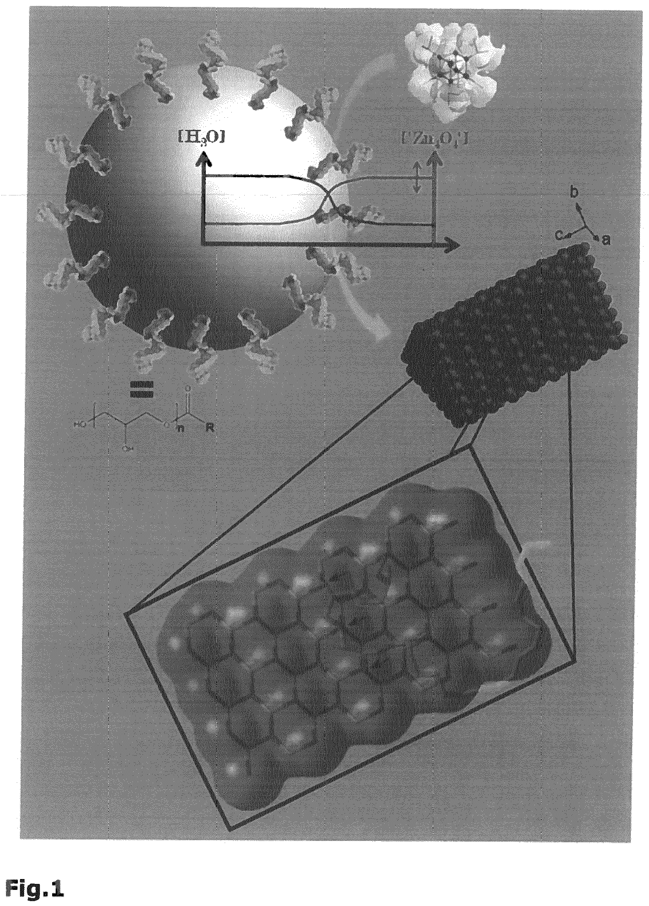

According to the invention the manufacturing of the ZnO occurs in an aprotic non-polar organic, non-coordinating solvent. The solvent itself is not able to interact with the surfaces of the forming inorganic phases and this enforces a much more directed interaction of the additive with the inorganic surfaces in comparison to the situation in coordinating solvents (e.g. water). Secondly, due to the limited solubility of ionic species in organic solvents any mass transport trough the continuous phase can be excluded. This means, that the growth process is restricted to the accretion of the monomeric species present in a supersatured solution. In the course the particles are prevented from growing large. The process of the invention is schematically depicted in Fig. 1.

One of the advantages of the process of the invention is that besides standard parameters like concentrations, temperatures, solvents etc. a number of additional parameters which might be important during the growth process of the inorganic phase can be adjusted:
In the process of the invention the emulsifier is preferably an amphiphile: The amphiphile used according to the invention fulfills simultaneously at least two tasks (see Fig. 1). Firstly, it stabilizes the emulsion droplets. Secondly, the amphiphile - headgroup interacts with the surfaces of the emerging ZnO phase. Therefore, an emulsifier system was selected in which the headgroup contains a number of functional groups disposed in a defined way. Surfactants with e.g. polyglyceryl head groups which can particularly be used, satisfy the above requirements as they potentially coordinating oxygen atoms in ether groups (C-O-C) and alcohol groups separated by a C-C bridge (see Fig. 1).

Figure 1: Schematical representation of the transformation of the 'Zn₄O₄' heterocubane precursors to ZnO due to a precursor and water concentration gradient at the interface of an emulsion droplet indicated in the graph. The emulsion droplet is stabilized by amphiphiles containing a polyglycery headgroup. A ZnO nanoparticle with prismatic morphology is shown and for better orientation the orientation of the lattice directions with respect to the elemental cell is indicated. The enlarged image demonstrates the structure of the unreconstructed {100}-surface with one amphiphile on top. It it is demonstrated that the distance of the oxygen-donor centers in the polyglyceryl fit ideally to the distance of the Zn²⁺ centers on the {100}-surface perpendicular to the c- direction (see arrows).

Further additives may be employed. Because the ZnO formation will take place at the interface of the emulsion droplet further growth effects might be evoked from additives dissolved either in the aqueous phase or the organic phase.

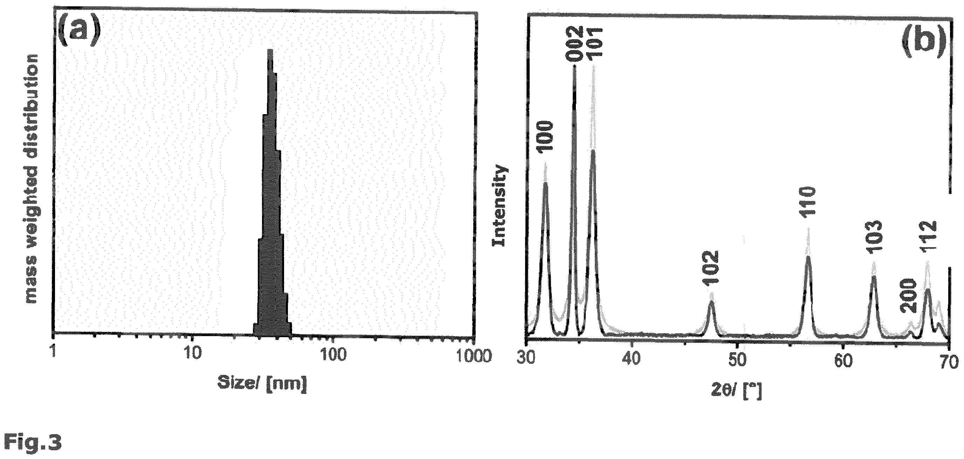

If the precursor concentration is increased further the stability of the emulsion in the course of the reaction may be impaired. The pastes are homogeneous and stable over time and at elevated temperature. The inspection of the materials via fluorescence microscopy indicated no phase separation or considerable aggregation. Interestingly, the samples showed a strong green luminescence. The stability of the samples is also documented by the fact that it was possible to re-disperse them quantitatively in various organic solvents like cyclohexane or tetrahydrofurane (THF). The resulting solutions were nearly transparent indicating that the described pastes represent a stable colloidal suspension of nanosized ZnO particles. Dynamic light scattering was determined on a colloidal solution obtained after re-dispersion of paste ZnO-2 in THF. The particle size distribution function is shown in Fig. 3a and indicates that fairly monodisperse particles with an average particle size of *Dₚ* ≈ 38 nm are present in materials ZnO-2.

According to the invention a precursor is used which is highly soluble in apolar media and which is able to release the inorganic material as desired. This precursor is the versatile, organometallic alkylzinc-alcoxy heterocubane precursor (denoted as 'Zn₄O₄') which is suitable for the generation of a large number of different ZnO materials. (S. Polarz, A. Roy, M. Lehmann, M. Driess, F. E. Kruis, A. Hoffmann, P. Zimmer, Adv. Funct. Mater. 2007, 17, 1385; S. Polarz, J. Strunk, V. Ischenko, M. Van den Berg, O. Hinrichsen, M. Muhler, M. Driess, Angew. Chem. 2006, 118, 3031; S. Polarz, F. Neues, M. Van den Berg, W. Grünert, L. Khodeir, J. Am. Chem. Soc. 2005, 127, 12028; C. Lizandara-Pueyo, M. van den Berg, A. de Toni, T. Goes, S. Polarz, J. Amer. Chem. Soc. 2008, 130, 16601; S. Polarz, A. Orlov, F. Schüth, A. H. Lu, Chem. Eur. J. 2007, 13, 592; M. Van den Berg, S. Polarz, O. P. Tkachenko, K. V. Klementiev, M. Bandyopadhyay, L. Khodeir, H. Gies, M. Muhler, W. Grünert, J. Catal. 2006, 241, 446; S. Polarz, A. Roy, M. Merz, S. Halm, D. Schröder, L. Scheider, G. Bacher, F. E. Kruis, M. Driess, Small 2005, 1, 540; V. Ischenko, S. Polarz, D. Grote, V. Stavarache, K. Fink, M. Driess, Adv. Funct. Mater. 2005, 15, 1945). This single-source precursor is a system which satisfies the requirements formulated above: It is soluble in a variety of aprotic, non-polar solvents, it can be converted to ZnO via hydrolysis, and it allows a fine control and tuning of kinetic parameters of crystallization. According to the process of the invention, in particular methylzinc-isopropoxide heterocubane is a suitable precursor.

The basic idea is that the formation of the ZnO originating from the 'Zn₄O₄' precursors dissolved in an aprotic non-polar solvent, such as a saturated, unsaturated or aromatic hydrocarbon, in particular a cyclic hydrocarbon such as cyclohexane, takes place in the water-oil interphase of emulsion droplets according to the reaction:

[CH₃ZnOCH(CH₃)₂]₄ ≡ 'Zn₄O₄' + 4 H₂O —> 4 ZnO + 4 CH₄ + 4 (CH₃)₂CHOH

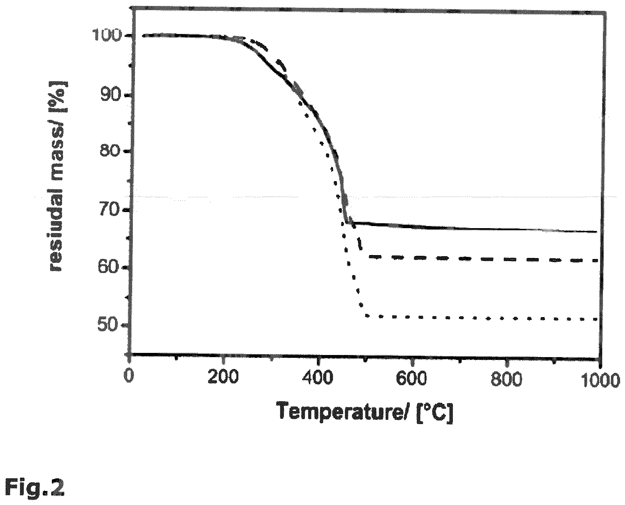

The rate of ZnO formation depends on the concentration c of the 'Zn₄O₄' precursor. Therefore, under otherwise fixed conditions c('Zn₄O₄') was varied systematically (see table 1). At the end of the reaction and after solvent removal one obtains colorless pastes. The viscosity of the pastes increases with increasing ZnO content. The ZnO content was determined by thermogravimetric analysis in air (20% O₂) shown in Fig. 2.

Figure 2: TGA traces for the samples ZnO-4 (dotted line), ZnO-5 (dashed line) and ZnO-7 (solid line).The nanoparticles of ZnO can either be directly used in the form of the dispersion as obtained from the process of manufacturing or the obtained colloidal dispersion is further purified to obtain purified nanoparticles of ZnO.

**Table 1: Overview over the samples prepared via the emulsion method. Further details are given in the examples.**

| **Sample-Code** | ***N(Zn₄O₄')*/ [mol]** | ***c('Zn₄O₄')l* [mol/l]** |
|---|---|---|
| **ZnO-1** | 1.2× 10⁻³ | 2.4×10⁻² |
| **ZnO-2** | 1.8×10⁻³ | 3.6×10⁻² |
| **ZnO-3** | 2.7×10⁻³ | 5.4×10⁻² |
| **ZnO-4** | 3.6×10⁻³ | 7.2×10⁻² |
| **ZnO-5** | 5.4×10⁻³ | 1.1×10⁻¹ |
| **ZnO-6** | 6.3×10⁻³ | 1.3×10⁻¹ |
| **ZnO-7** | 7.2×10⁻³ | 1.4×10-1 |

Figure 3: (a) Particle size distribution function determined from DLS measurements performed on a colloidal solution of sample ZnO-2 redispersed in THF. (b) PXRD data of sample ZnO-2 (black curve) in comparison to a ZnO material prepared without the of an polyglyceryl containing amphiphile as a reference (grey curve).

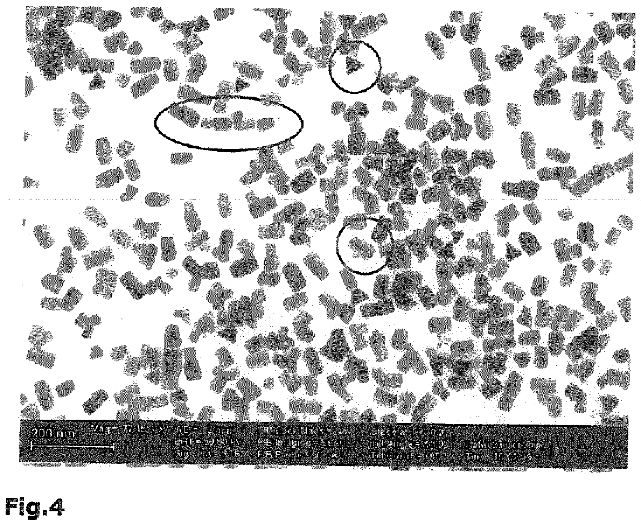

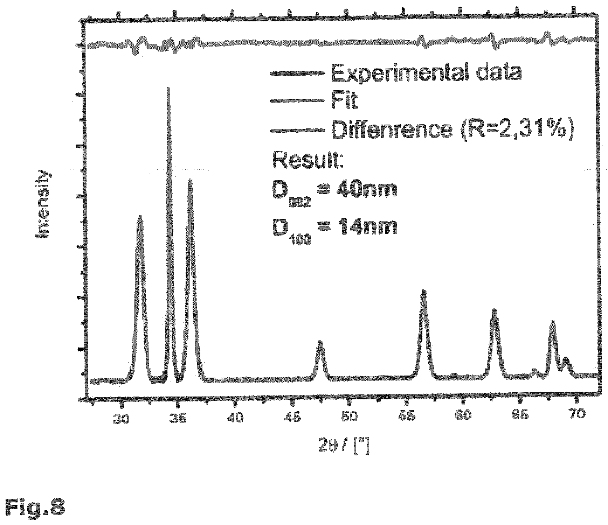

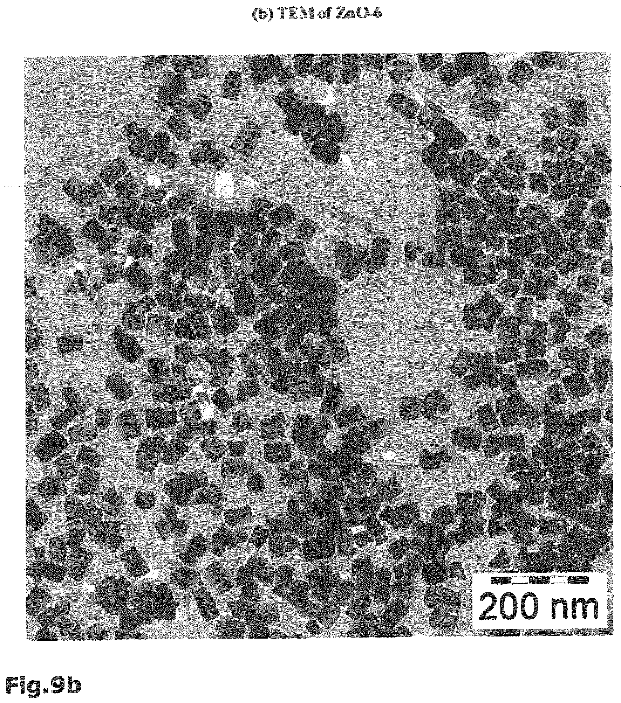

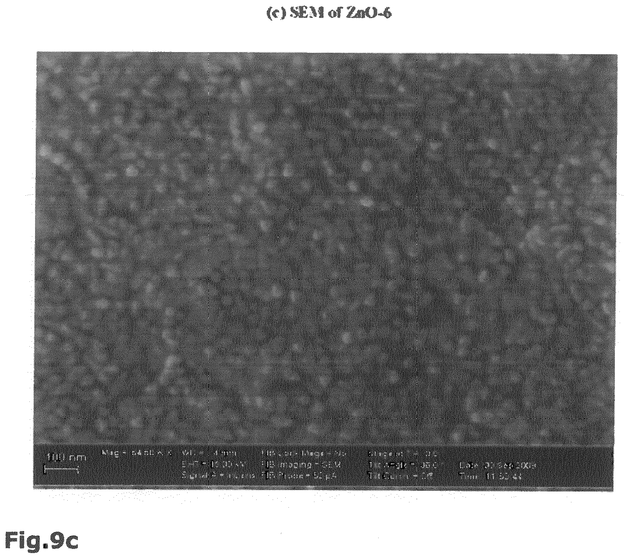

The crystallinity of ZnO-2 was investigated by powder x-ray diffraction (PXRD) shown in Fig. 3b in comparison to a reference sample prepared in the absence of a polyglyceryl containing amphiphile. As expected zinc oxide in the Wurtzite modification is found. However, the PXRD pattern of ZnO-2 is strikingly different from the pattern of "common" zinc oxide. The most distinct difference is that the reflex corresponding to the {002} lattice plane is more narrow and more intense than expected. All other reflexes, in particular the (101) and (110) reflexes are less intense than for the reference sample. The described results can be explained if the particles in the ZnO-2 sample are anisotropic with a preferential orientation along the {002} plane. A refined analysis of the PXRD data was performed using a Warren-Averbach analysis. The refined evaluation confirms that the particles have nanoscale dimensions with *D_{P}* = 35nm in [002] direction and *D_{P}* = 17nm in the directions perpendicular to [002] (see Fig. 8). The particle dimension determined from PXRD are obviously in good agreement with the DLS measurements. It should be noted that ZnO with nanorod morphology with a hexagonal basis has already been reported by several groups in the past. For instance, Chaudret et al. have reported about the formation of ZnO nanorods obtained from the reaction of dicyclohexylzinc in THF with air (M. L. Kahn, T. Cardinal, B. Bousquet, M. Monge, V. Jubera, B. Chaudret, Chemphyschem 2006, 7, 2392; M. L. Kahn, M. Monge, V. Colliere, F. Senocq, A. Maisonnat, B. Chaudret, Advanced Functional Materials 2005, 15, 458; M. Monge, M. L. Kahn, A. Maisonnat, B. Chaudret, Angewandte Chemie-International Edition 2003, 42, 5321). For the elucidation of the exact morphology of the ZnO particles a number of analytical techniques was applied: Transmission electron microscopy (TEM; shown in FIG. 9b), scanning electron microscopy (SEM; shown in FIG. 9c) and scanning transmission electron microscopy (STEM). In particular the STEM images are illuminating (see Fig. 4: STEM image of sample ZnO-4).

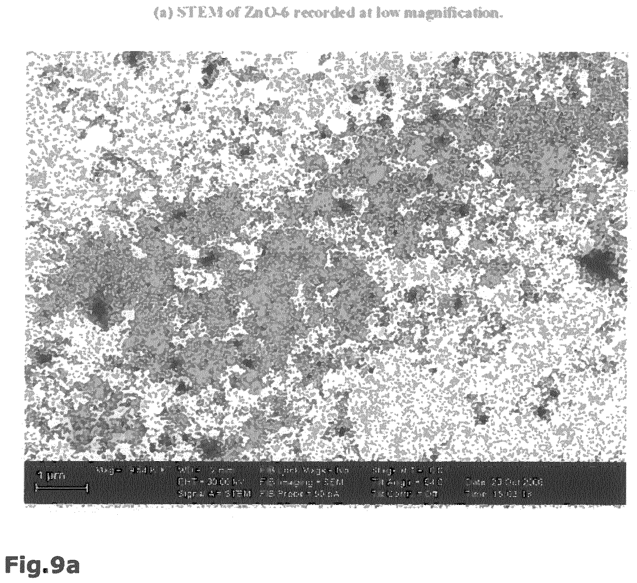

In addition to the elongated shape of the particles three characteristic features can be identified. Many particles display a dark stripe on top as if they were thicker in the middle than at the sides. The latter observation is supported by the assumption that the basis of the particles is triangular. The particles tend to form chain-like structures. Because the {002} surfaces are the polar surfaces in the Wurtzite crystal structure, and because from PXRD it is known that the particles are elongated in the [002] direction, the latter observation can be explained with the fact that each particle possesses a dipole moment along the c-axis. The sum over all the shown analytical techniques is consistent only with one special morphology of ZnO, a nanoprism shape as shown in Fig. 1. Images recorded at lower magnifications indicate that the vast majority of the sample is characterized by particles possessing the described prismatic morphology (see FIG. 9a).

According to the invention the emulsifier is in particular an emulsifier binding to the {100} lattice plane of Zn, in particular polyglyceryl-3 polyricinoleate or other polyglyceryl containing stabilizers like polyglyceryl-(4)-isostearat etc.

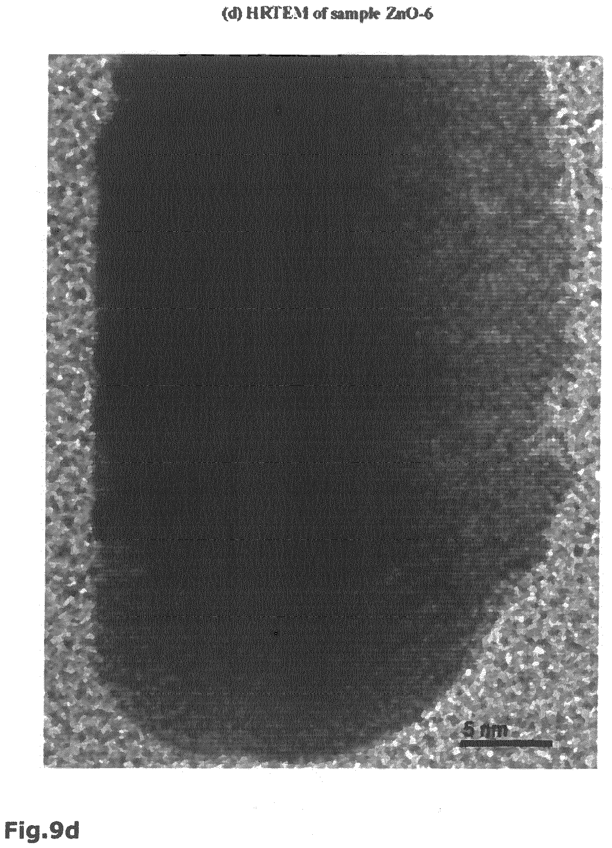

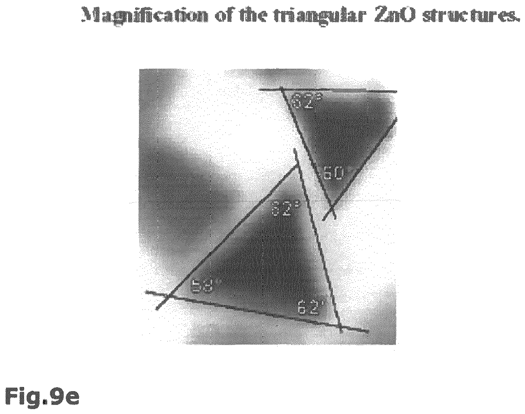

An angle of 60° can be identified at the triangular structures (see FIG. 9e). There is only one set of symmetry equivalent surfaces which are perpendicular to the {002} lattice plane and at the same time and are oriented in an angle of 60° to each other. These are the surfaces (100), (010) and (-110). The lateral surfaces of the ZnO nanoprism correspond to the (100) lattice plane, as shown by HRTEM images (see FIG. 9d).

Although not being bound by any hypotheses, it is believed that the described shape of the nanoparticles can be explained by selective interaction of e.g. the polyglyceryl group with the (100) surface during the growth of the ZnO phase. Then, the {100} surfaces would have been specifically blocked against apposition of ZnO, growth is directed to the [002] direction. A comparison between the geometric structure of the unreconstructed {100} ZnO surfaces and the polyglyceryl headgroup indicates a quite good match. The distance of coordinatively unsaturated Zn²⁺ centers (coordination number 3) is 3.3 Å in comparison to the distance of the ether and alcohol oxygen atoms of 3.4 Å in the polyglyceryl block determined from force-field calculations.

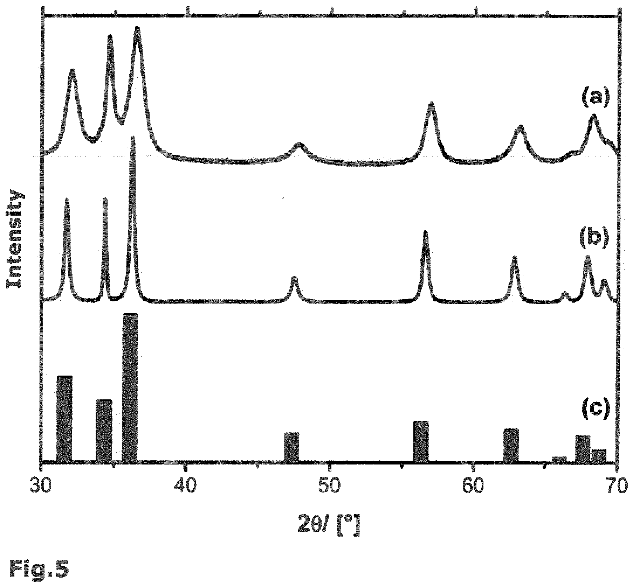

Instead of polyglyceryl-3-polyricinoleate alternative emulsifiers can be used for the synthesis. The PXRD patterns of a sample prepared with polyglyceryl-4-oleate and a sample prepared with Triton®-X are compared to each other in Fig. 5.

Figure 5: PXRD pattern of ZnO particles prepared via the emulsion method with polyglyceryl-2-oleate (a) and Triton®-X as an amphiphile. In (c) the pattern of single- crystalline ZnO (JCPD file 79-0208 from the ICCD x-ray database) is shown as a reference.

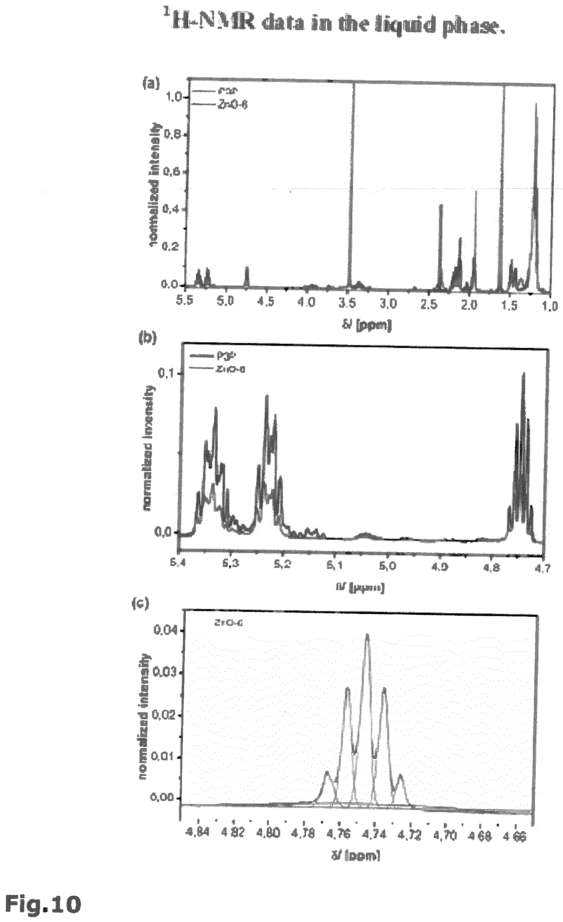

Although Triton®-X contains a non-ionic poly-ethylene oxide head group which is very similar to the poly-glyceryl headgroup there is no unusual narrowing of the (002) reflex visible and the corresponding PXRD pattern (Fig. 5b) looks very similar to the pattern expected for single-crystalline ZnO (Fig. 5c) indicating the formation of particles with isotropic shape. The situation changes for polyglyceryl-4-oleate. Not only that the particles are smaller, in addition similar changes are present which have been described previously for the synthesis utilizing polyglyceryl-3-polyricinoleate. The latter assumption is furthermore supported by ¹H-NMR measurements recorded in solution (spectra shown in Fig. 10). It can be seen from the spectra that in particular the NMR-signal related to the polyglyceryl entitity (δ = 4.7-5.4 ppm) possesses significantly lower integrated intensity in sample ZnO-6 in comparison to the unbound polyglyceryl surfactant as a reference. The decrease in intensity can be rationalized as follows: The interaction between the polyglyceryl entities and the ZnO surface leads to a decrease in mobility. This in turn causes a significant broadening of the corresponding signals. In ¹H-NMR only the groups with sufficient mobility are visible. The latter results clearly substantiate that the polyglyceryl head group is a deciding factor regarding the formation of the anisotropic, prismatic ZnO nanostructures.

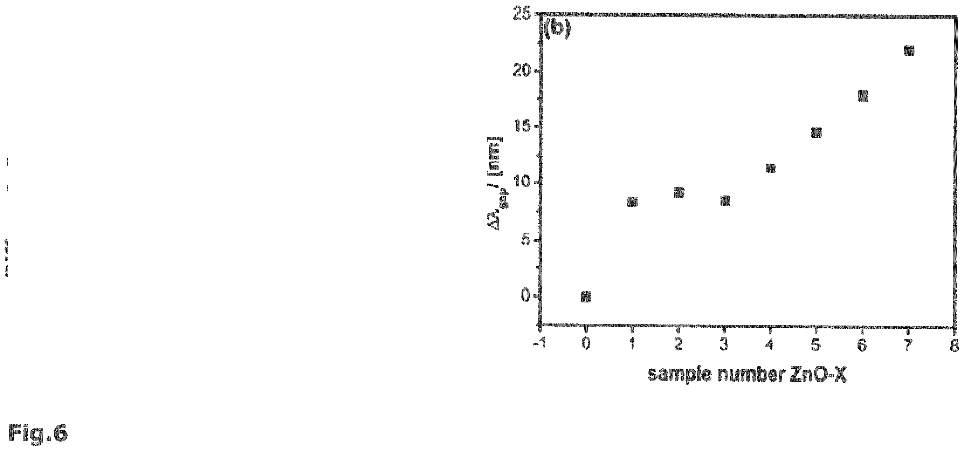

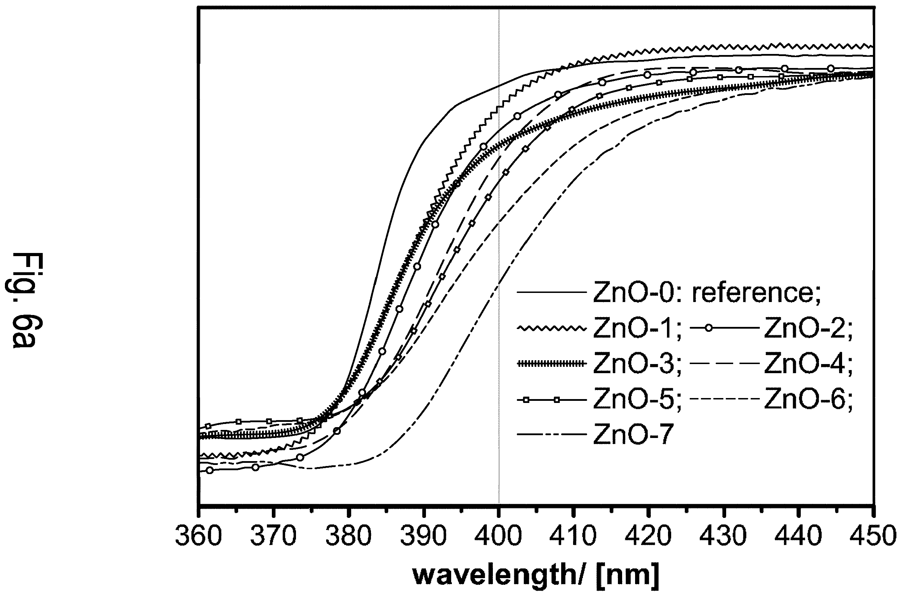

UV/Vis- spectroscopy was applied for the investigation of the optical properties of the obtained ZnO materials (Fig. 6).

Figure 6: (a) UV/Vis spectra of the samples ZnO-1 through ZnO-7, and of a reference zinc oxide sample ZnO-0. The line at 400 nm implies the extension of the UV-A region (315-400nm) in the electromagnetic spectrum. (b) The shift of the band-gap energy Δλ_{gap} of the samples ZnO-1—>7 is shown in comparison to the reference ZnO-0 (Δλ_{gap=} 0nm).

In comparison to a reference zinc oxide sample (ZnO-0) prepared without a polyglyceryl additive it is seen that the band-gap energies of the samples ZnO-1-7 are significantly shifted to lower energies (Fig. 6a). Interestingly, the higher the concentration of the ZnO precursor has been during the preparation of the materials (see table 1) the stronger is the red-shift. The latter correlation is shown in Fig. 6b. The described findings are surprising because the band-gap energy is supposed to be a property which depends on the size (volume) of the crystalline particles. Above a particle size of ≈ 10nm quantum confinement effects should be of negligible importance and anyhow it would lead to a blue-shift of the energy gap not vice versa. Although in principle each particular surface (hkl) possesses its own characteristic electronic system, the contribution of the surface characteristic with respect to the electronic system of the particles is rather small for particle sizes above 10nm. However, the results presented in Fig. 6 clearly indicate that the unusual prismatic morphology of the zinc oxide nanoparticles in the samples ZnO-1—>7 is related to the red-shift of the band-gap.

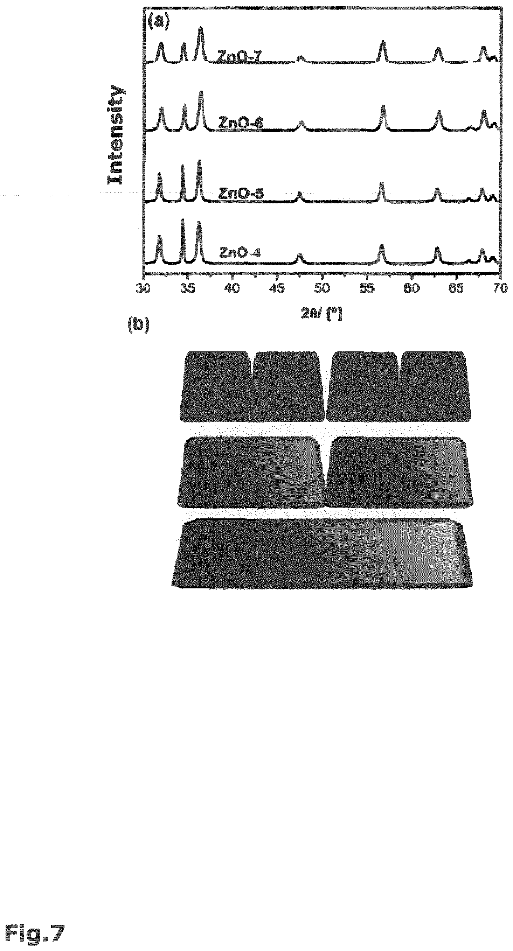

Up to date, there is no explanation for the surprising differences of band-gap shifts between the samples. Therefore, the samples which showed a significant shift of the band-gap (ZnO-4—>7) were also investigated with PXRD shown in Fig. 7a.

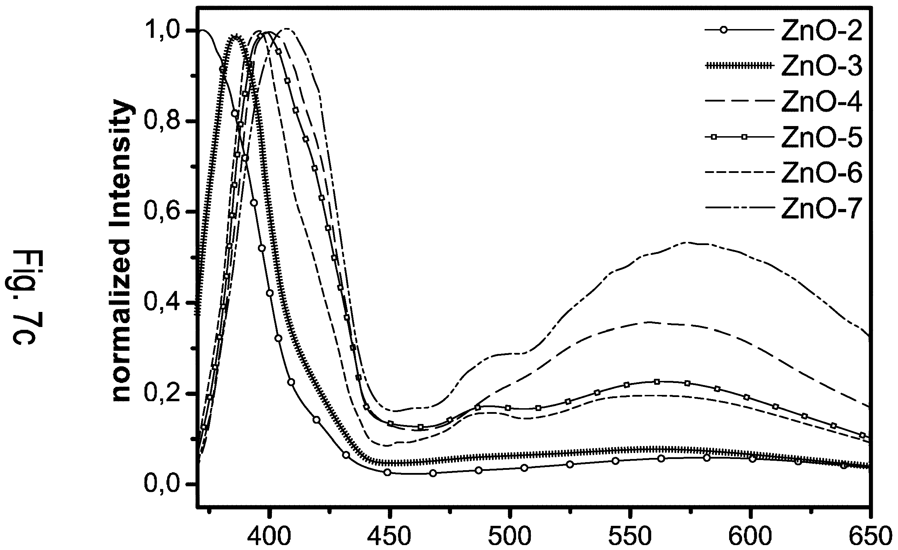

Figure 7: (a) PXRD patterns of the ZnO-4—>7 samples. (b) Illustration that shorter ZnO nanoprisms result in a larger number of (002)-(100) edges ([002] plotted in green, [100] plotted in blue). (c) Photoluminescence spectra of samples ZnO-2—>7.

Surprisingly, it is not the particle anisotropy alone which is responsible for the changes in optical properties. The sample containing the shortest ZnO nanoprisms (ZnO-7) as seen from the systematic decrease of the intensity of the (002) reflex (Fig. 7a) is the one with the band-gap of lowest energy (see Fig. 6). We know from our experiences with the used precursor system that a higher concentration of precursor (see table 1) leads to higher supersaturation which in turn leads to an increased number of critical nuclei. The consequence of a very large number of critical nuclei is that during simultaneous growth conditions each single particle remains relatively small. Therefore, it is reasonable that in the sequence ZnO- 1 —>7 the particles become shorter.

It is believed that two factors contribute to the remarkable optical properties. The special edge created by the (100) and the (002) surface is responsible for the finding that the shortest nanoprisms display the strongest optical changes. It can be seen from Fig. 7b that the shortage of the ZnO nanoprisms leads to an enhancement of the number of (100)-(002) edges in a linear fashion similar to the linear dependency observed for Δλ_{gap} seen for the samples ZnO-3—>7 in Fig. 6b. The hypothesis is that due to the low coordination degree of the atoms at the (100)-(002) edge local defect formation is induced, and that the number of these defects is responsible for the extent of the red-shift of the band-gap energy. This assumption is supported by photoluminescence spectroscopy (see Fig. 7c). Furthermore, one has to consider that the {002} surfaces are the so-called polar surface of ZnO. The prismatic ZnO particles possess a dipole moment because one (002) surface contains only Zn²⁺ ions and the opposite surface only O²⁻ ions. For short particles the electric field created by the dipole moment will effect the electronic system of the entire particle. This effect on the electronic system can also induce a decrease of the band-gap of the system.

Two fluorescence signals can be observed for the ZnO samples prepared in the current study. In the region 390-425nm a broad photoluminescence signal for the transition at the band-gap can be seen. The latter transitions are shifted towards lower energy compared to the photoluminescence of conventional ZnO (not shown). However, in addition to the mentioned signal an even wider luminescence signal in the spectral region 500-650nm can be detected. This luminescence, also known as the so-called green luminescence, has been related in the literature to the density of defects in ZnO. The intensity of the band-gap luminescence was normalized to 1 for better comparison between the different samples. It is evident that the intensity of the green luminescence and thus the defect density increases in the order ZnO-2—>7.

Subject matter of the invention is also a colloidal ZnO with prismatic shape having a triangular basis containing preparation obtainable by the process of the invention. This ZnO is used due to its described red-shift as UV-protective material for cosmetic and industrial applications.

The invention is further described by the following non-limiting examples.

### Examples

All starting compounds were purified and carefully dried prior to use. All reactions were performed under strict exclusion of air and humidity using Schlenck technique.

### Preparation of ZnMe₂:

ZnCl₂ is dried via the treatment with SOCI2 under reflux. Remaining SOCI2 is removed in vacuum. A solution of CH₃MgI (1.25 mol) in n-Bu₂O is slowly added to the ZnCl₂ (0.57 mol) under vigorous stirring. The suspension is heated for 60h at 60°C under reflux. The ZnMe₂ (b.p. = 43°C) is collected by distillation in the temperature range 130°-160°C. 40.8g ZnMe₂ is obtained in 75% yield.

### Preparation of the precursors [MeZnO^{iso}Pr]₄:

ZnMe₂ is diluted with freshly dried and distilled toluene. The solution is cooled to -78°C. An equimolar amount of isopropanol in toluene is added drop wise.

The solution is warmed to RT and stirred for 12h. The toluene is removed in vacuum. The resulting white solid is taken up in dry hexane. Residual solid products are separated by Schlenck centrifugation. The pentane is removed from the solution by vacuum drying. The product is obtained in quantitative yield. The purity of the product is checked by NMR spectroscopy. [MeZnO^{iso}Pr]₄ is kept inside a glove-box until use.

¹H-NMR (400 MHz, CDCl3): *δ* / [ppm] = -0.63 (s, 3H, Zn-CH₃); 1.24 (d, 6H, ³J = 7.1 Hz, CH(CH₃)₂); 4.13 (sept., 1H, ³J = 6.1 Hz, CH(CH₃)₂).

¹³C-NMR (400 MHz, CDCl₃): *δ* / [ppm] = -12.44 (Zn-CH₃); 27.00 (CH(CH₃)₂); 68.87 (CH(CH₃)₂).

### Emulsion synthesis of colloidal ZnO dispersions:

Water (1 ml), cyclohexane (35 ml) and a polyglyceryl-containing emulsifier like polyglyceryl-3-polyricinoleate (1 ml) are mixed. An emulsion is prepared by applying ultrasound for 15 min using a Bandelin-Sonoplus TT13/FZ ultrasonification device. The emulsion was heated at 40°C. Different amounts of [MeZnO^{i-so}Pr]₄ (see table 1) are dissolved in dry cyclohexane (15 ml) and added dropwise to the emulsion. During addition and for additional 60 min ultrasonification of the emulsion is continued. The samples are kept at T = 115°C inside a drying oven for removing residual water.

## Claims

1. A process for manufacturing colloidal dispersions of ZnO comprising the steps of:
- providing an emulsion of an aqueous solution, an aprotic non-polar organic solvent which is essentially non-soluble with water, and an emulsifier containing functional groups capable of specific interaction with ZnO surfaces selected from the group consisting of a polyglycerylate;
- adding, by maintaining the emulsion, as organo metallic precursor a tetrameric zinc-oxo compound selected from the group consisting of the formula [RZnOR']₄ where R is Me or Et and R' is Me, Et, Pr, Bu, iso-Pr, tert-Bu, or OCH₂CH₂OCH₃, able to form ZnO;
- reacting the mixture for a sufficient time to form ZnO,
- removal of the water and the aprotic non-polar organic solvent upon forming of said ZnO, and
- isolating the reaction product in which nanoparticles of ZnO with prismatic shape having a triangular basis are obtained in a colloidal dispersion.

2. The process of claim 1 wherein the non-polar organic solvent is a saturated, unsaturated or aromatic hydrocarbon.

3. The process of claim 2 wherein the emulsifier is polyglyceryl-polyricinoleate.

4. The process of any one of the claims 1 to 3 wherein the metallo organic ZnO precursor is methylzinc-isopropoxide heterocubane.

5. The process of any of the claims 1 to 4, wherein the metallo organic ZnO precursor is a single-source precursor.

6. A colloidal ZnO with a prismatic shape having a triangular basis containing preparation obtainable by the process according to the combination of claims 3 and 4.

7. Use of the ZnO preparation of claim 6 as UV-A absorbing agent.

8. Use of the ZnO particles of claim 6 as UV-protective material for cosmetic and industrial applications.

## Patentansprüche

1. Verfahren zur Herstellung kolloidaler Dispersionen von ZnO, umfassend die Schritte:
- Bereitstellen einer Emulsion einer wässrigen Lösung, eines aprotischen unpolaren organischen Lösungsmittels, das mit Wasser im Wesentlichen nicht mischbar ist, und eines Emulgators, der funktionelle Gruppen enthält, die zu einer spezifischen Wechselwirkung mit ZnO-Oberflächen befähigt sind, und der aus der Gruppe ausgewählt ist, die aus einem Polyglycerylat besteht;
- Hinzufügen einer tetrameren Zink-Oxo-Verbindung, die aus der Gruppe ausgewählt ist, die aus der Formel [RZnOR']₄ besteht, wobei R = Me oder Et ist und R' = Me, Et, Pr, Bu, iso-Pr, tert-Bu oder OCH₂CH₂OCH₃ ist, und die ZnO bilden kann, als metallorganischer Vorläufer unter Aufrechterhalten der Emulsion;
- Umsetzen des Gemischs während einer ausreichenden Zeit unter Bildung von ZnO;
- Entfernen des Wassers und des aprotischen unpolaren organischen Lösungsmittels bei der Bildung des ZnO; und
- Isolieren des Reaktionsprodukts, wobei Nanopartikel von ZnO in Form von Prismen mit dreieckiger Basis in kolloidaler Dispersion erhalten werden.

2. Verfahren gemäß Anspruch 1, wobei das unpolare organische Lösungsmittel ein gesättigter, ungesättigter oder aromatischer Kohlenwasserstoff ist.

3. Verfahren gemäß Anspruch 2, wobei es sich bei dem Emulgator um Polyglycerylpolyricinoleat handelt.

4. Verfahren gemäß einem der Ansprüche 1 bis 3, wobei es sich bei dem metallorganischen ZnO-Vorläufer um Methylzinkisopropoxid-Heterocuban handelt.

5. Verfahren gemäß einem der Ansprüche 1 bis 4, wobei der metallorganische ZnO-Vorläufer ein Einzelquellenvorläufer ist.

6. Präparat, das kolloidales ZnO in Form von Prismen mit dreieckiger Basis enthält und nach dem Verfahren gemäß einer Kombination der Ansprüche 3 und 4 erhältlich ist.

7. Verwendung des ZnO-Präparats gemäß Anspruch 6 als UV-A-Absorptionsmittel.

8. Verwendung der ZnO-Teilchen gemäß Anspruch 6 als UV-Schutzmaterial für kosmetische und industrielle Anwendungen.

## Revendications

1. Procédé pour fabriquer des dispersions colloïdales de ZnO comprenant les étapes consistant à :
- procurer une émulsion d'une solution aqueuse, un solvant organique non polaire aprotique qui est sensiblement insoluble avec de l'eau, et un émulsifiant contenant des groupes fonctionnels capables d'une interaction spécifique avec des surfaces de ZnO, choisi dans le groupe consistant en un polyglycérylate,
- ajouter, en maintenant l'émulsion, comme précurseur organométallique un composé zinc-oxo tétramérique choisi dans le groupe consistant en la formule [RZnOR']₄, où R est Me ou Et et R' est Me, Et, Pr, Bu, iso-Pr, tert-Bu ou OCH₂CH₂OCH₃, capable de former du ZnO,
- faire réagir le mélange pendant un temps suffisant pour former du ZnO,
- prélever l'eau et le solvant organique non polaire aprotique au moment de la formation dudit ZnO, et
- isoler le produit de réaction dans lequel des nanoparticules de ZnO avec une forme prismatique ayant une base triangulaire sont obtenues dans une dispersion colloïdale.

2. Procédé selon la revendication 1, dans lequel le solvant organique non polaire est un hydrocarbure saturé, insaturé ou aromatique.

3. Procédé selon la revendication 2, dans lequel ledit émulsifiant est le polyglycéryl-polyricinoléate.

4. Procédé selon l'une quelconque des revendications 1 à 3, dans lequel le précurseur de ZnO organométallique est l'hétérocubane isopropoxyde de méthyl-zinc.

5. Procédé selon l'une quelconque des revendications 1 à 4, dans lequel le précurseur de ZnO organométallique est un précurseur à source unique.

6. Préparation contenant du ZnO colloïdal avec une forme prismatique ayant une base triangulaire, pouvant être obtenue par le procédé selon une combinaison des revendications 3 et 4.

7. Utilisation de la préparation de ZnO selon la revendication 6 comme agent absorbant les UV-A.

8. Utilisation des particules de ZnO selon la revendication 6 comme matériau protecteur UV pour des applications cosmétiques et industrielles.
